# EUROPÄISCHE PATENTANMELDUNG

(11) **EP 2 241 352 A2**
(43) Veröffentlichungstag der Anmeldung: **20.10.2010**
(21) Anmeldenummer: 10156451.6
(22) Anmeldetag: 15.03.2010
(51) Int. Cl.: A61Q 15/00, A61K 8/86, A61K 8/39, C08G 65/26

(54) **Verfahren zur Herstellung von geruchlosen Polyetheralkoholen mittels DMC-Katalysatoren und deren Verwendung in kosmetischen und/oder dermatologischen Zubereitungen**

(30) Priorität: 15.04.2009 DE 102009002371
(71) Anmelder: Evonik Goldschmidt GmbH, 45127 Essen (DE)
(72) Erfinder: Springer, Oliver, 46485, Wesel (DE); Thum, Oliver, 40880, Ratingen (DE); Schubert, Frank, 47506, Neukirchen-Vluyn (DE)

(57) **Zusammenfassung**

Kosmetische und/oder dermatologische Zubereitungen hergestellt unter Verwendung von Polyetheralkoholen, **dadurch gekennzeichnet, dass** zur Herstellung der Polyetheralkohole
a) ein DMC-Katalysator verwendet wird und
b) die so erhaltenen Polyetheralkohole nicht weiter behandelt werden.

## Beschreibung

Polyetheralkohole, oft auch kurz einfach als Polyether bezeichnet, sind seit langem bekannt und werden in großen Mengen technisch hergestellt. Sie dienen unter anderem als Tenside, Emulgatoren oder Schaumdämpfer. Die gewünschten Eigenschaften lassen sich dabei gezielt durch die Art des Alkohols und die Art und Menge des Polyetheranteils einstellen. Reine Propylenoxid basierende Polyetheralkohole werden auch als Ölphase in kosmetischen Zubereitungen, u.a. AP/Deo Formulierungen (= Antiperspirant/Desodorant Formulierungen) eingesetzt.

Die meisten Verfahren zur Herstellung von Alkoxylierungsprodukten (Polyethern) bedienen sich basischer Katalysatoren wie z.B. der Alkalihydroxide und der Alkalimethylate. Besonders verbreitet und seit vielen Jahren bekannt ist der Einsatz von KOH oder NaOH. Typischerweise wird ein meist niedermolekularer hydroxy-funktioneller Starter (Startalkohol) wie Butanol, Allylalkohol, Propylenglykol oder Glycerin in Gegenwart des alkalischen Katalysators mit einem Alkylenoxid wie Ethylenoxid, Propylenoxid, Butylenoxid oder einem Gemisch verschiedener Alkylenoxide zu einem Polyoxyalkylenpolyether umgesetzt. Die stark alkalischen Reaktionsbedingungen bei dieser sogenannten Living-Polymerisation fördern verschiedene Nebenreaktionen. Nachteilig sind vor allem die aufwendigen Produktaufarbeitung bedingt durch Neutralisation des alkalischen Polymerisates (siehe z.B. US 3,715,402, US 4,430,490, US 4,507,475 und US 4,137,398) und die als Nebenreaktion ablaufende basenkatalysierte Umlagerung von Epoxiden, beispielsweise Propylenoxid, zu Allyl- bzw. Propenylalkoholen. Diese Propenylpolyether erweisen sich in kosmetischen Zubereitungen - durch die hydrolytische Labilität der in ihnen enthaltenen Vinyletherbindung und Freisetzung von Propionaldehyd - als unerwünschte Quelle olfaktorischer Produktbelastungen. Ein Verfahren zur Herstellung geruchsarmer Polyetherpolyole ist u.a. in EP 1 062 263 beschrieben.

Zu den Nachteilen der basisch katalysierten Alkoxylierung zählt zweifelsfrei die Notwendigkeit, die erhaltenen Reaktionsprodukte mit Hilfe eines Neutralisationsschrittes von der aktiven Base zu befreien. Zwingend erforderlich sind dann die destillative Abtrennung des bei der Neutralisation entstehenden Wassers sowie die Abtrennung des gebildeten Salzes durch Filtration.

Neben der basenkatalysierten Reaktion sind auch saure Katalysen zur Alkoxylierung bekannt. So wird in DE 10 2004 007561 die Verwendung von HBF₄ und von Lewis-Säuren wie z.B. BF₃, AlCl₃ und SnCl₄ in der Alkoxylierungstechnologie beschrieben.

Nachteilig bei der säurekatalysierten Polyethersynthese erweist sich die mangelhafte Regioselektivität bei der Ringöffnung unsymmetrischer Oxirane wie z.B. Propylenoxid, die dazu führt, dass in nicht eindeutig zu steuernder Weise Polyoxyalkylenketten mit teils sekundären und primären OH-Termini erhalten werden. Wie im Falle der basenkatalysierten Alkoxylierungsreaktion ist auch hier eine Aufarbeitungssequenz von Neutralisation, Destillation und Filtration unabdingbar. Wird Ethylenoxid als Monomer in die säurekatalysierte Polyethersynthese eingebracht, so ist mit der Bildung von Dioxan als unerwünschtem Nebenprodukt zu rechnen.

Als Katalysatoren zur Herstellung von Polyetheralkoholen werden aber auch häufig Multimetallcyanidverbindungen oder Doppelmetallcyanid-Katalysatoren, allgemeingebräuchlich auch als DMC-Katalysatoren bezeichnet, eingesetzt. Durch den Einsatz von DMC-Katalysatoren wird der Gehalt an ungesättigten Nebenprodukten minimiert, außerdem verläuft die Umsetzung, verglichen mit den üblichen basischen Katalysatoren, mit einer deutlich höheren Raum-Zeit-Ausbeute. Die Herstellung und Verwendung von Doppelmetallcyanid-Komplexen als Alkoxylierungskatalysatoren ist seit den 1960er Jahren bekannt und wird zum Beispiel in US 3,427,256, US 3,427,334, US 3,427,335, US 3,278,457, US 3,278,458, US 3,278,459 dargestellt. Unter den in den Folgejahren weiter entwickelten und z.B. in US 5,470,813 und US 5,482,908 beschriebenen, immer wirksameren Typen von DMC-Katalysatoren befinden sich im speziellen Zink-Cobalt-Hexacyanokomplexe. Dank ihrer außerordentlich hohen Aktivität werden zur Herstellung von Polyetherolen nur geringe Katalysatorkonzentrationen benötigt, so dass auf die für konventionelle alkalische Katalysatoren notwendige Aufarbeitungsstufe - bestehend aus der Neutralisation, der Fällung und der Abfiltration des Katalysators - am Ende des Alkoxylierungsprozesses verzichtet werden kann. Die mit DMC-Katalysatoren hergestellten Alkoxylierungsprodukte zeichnen sich durch eine viel engere Molmassenverteilung im Vergleich zu alkalisch katalysierten Produkten aus. Auf die hohe Selektivität der DMC-katalysierten Alkoxylierung ist zurückzuführen, dass zum Beispiel Propylenoxid-basierende Polyether nur sehr geringe Anteile ungesättigter Nebenprodukte enthalten.

Die im unmittelbaren Vergleich mit Alkali- und Säurekatalyse an DMC-Katalysatoren durchgeführte Alkoxylierungsreaktion ist unter den beschriebenen technischen Eigenarten so vorteilhaft, dass sie zur Entwicklung kontinuierlicher Prozesse zur Herstellung volumenstarker einfacher, zumeist nur aus PO-Einheiten bestehender, Polyetherole geführt hat.

So beschreibt WO 98/03571 ein Verfahren zur kontinuierlichen Herstellung von Polyetheralkoholen mittels DMC-Katalysatoren, bei dem in einem kontinuierlichen Rührkessel zunächst eine Mischung aus einem Starter und einem DMC-Katalysator vorgelegt, der Katalysator aktiviert und zu dieser aktivierten Mischung kontinuierlich weiterer Starter, Alkylenoxide und DMC-Katalysator gegeben und, nach Erreichen des angestrebten Füllstands des Reaktors, kontinuierlich Polyetheralkohol abgezogen wird.

In E P 1 756 198 wird ein Verfahren zur Herstellung von geruchsarmen Polyetherpolyolen mittels DMC-Katalyse beschrieben, wobei die Polyether noch mittels Strippgas behandelt werden.

In WO 01/62826, WO 01/62824 und WO 01/62825 werden spezielle Reaktoren für das kontinuierliche Verfahren zur Herstellung von Polyetheralkoholen mittels DMC-Katalysatoren beschrieben.

Die Patentliteratur stellt bei den hier beschriebenen technischen Verfahren insbesondere auf die Monodispersität des nach DMC-Verfahren gewonnenen Polyetherols ab. So sind enge Molmassenverteilungen oft wünschenswert, wie im Falle der für PU-Verschäumungssysteme genutzten Polyole (DE 100 08630, US 5,689,012).

Nicht in allen Anwendungsgebieten ist eine geringe Molmassenverteilung jedoch gleichbedeutend mit hoher Qualität. In sensiblen Applikationen kann eine zu geringe Polydispersität sogar von Nachteil sein, was die Verwendbarkeit DMC-basierender Polyether/Polyetheralkohole einschränkt. So weist die Schrift EP-A-1066334 in diesem Zusammenhang darauf hin, dass man die nach alkalischem Alkoxylierungsverfahren gewonnenen Polyetheralkohole nicht in einfacher Weise durch die mittels DMC-Katalyse hergestellten Polyetherole substituieren kann. Einschränkung erfährt die Nützlichkeit der via DMC-Katalyse gewonnenen und durch ihre enge Molekulargewichtsverteilung charakterisierten Polyetheralkohole insbesondere dort, wo man sie als eine Komponente in einem Mehrkomponentensystem einsetzt. Dieses Mehrkomponentensystem kann u.a. eine kosmetische Zubereitung sein.

Beschreibung der Erfindung:
Aufgabe der Erfindung ist es, Rohstoffe für kosmetische und/oder dermatologische Zubereitungen zur Verfügung zu stellen, die bekannte Rohstoffe zumindest zu ersetzen vermögen und dabei die Stabilitäts- und olfaktorischen Probleme der Rohstoffe des Standes der Technik nicht mehr aufweisen.
Dies gilt besonders für kosmetische und/oder dermatologische Zubereitungen der Kategorie Antiperspirantien/Desodorantien, insbesondere in so genannten "Stick-Formulierungen", die oft noch das Problem der unzureichenden Stabilität zeigen, insoweit dass sich geruchliche Veränderungen während der Lagerung ergeben.

Überraschender Weise wurde gefunden, dass Polyetherole, die mittels DMC-Katalyse bei sehr geringen Katalysatorkonzentrationen hergestellt werden, ohne weitere Behandlungsschritte in kosmetischen und/oder dermatologische Zubereitungen, insbesondere AP/Deo Zubereitungen, eingesetzt werden können, ohne unerwünschte Geruchsentwicklungen, auch nach längerer Lagerzeit, zu zeigen.

Es gelingt mit den erfindungsgemäßen Zubereitungen der Ersatz bekannter Polyetherole, die durch säure- oder basenkatalysierte Verfahren hergestellt wurden und die Nebenprodukte aufweisen, die in aufwändiger Weise mittels Neutralisations-, Filtrations- und/oder Destillationschritten entfernt werden müssen.

Die nach dem DMC-Verfahren (Doppelmetallcyanid-Verfahren zur Alkoxylierung) hergestellten Polyetherole weisen diese Nachteile nicht mehr auf, können direkt ohne Aufreinigung und Neutralisation verwendet werden und können im direkten Ersatz gegen die bekannten Verbindungen ausgetauscht werden.

Mit dem DMC-Verfahren können je nach eingesetztem Epoxid und Art der Epoxidringöffnung Zusammensetzungen enthaltend Polyetheralkohole der Formel (I) hergestellt werden:

R¹-[O-(A)ₙ-(B)ₘ-H]ₓ (I)

wobei
- R¹: ein linearer oder verzweigter Alkylrest mit 3 bis 22 C- Atomen ist,
- A: unabhängig von B eine gleiche oder unterschiedliche Ethylenoxy-, Propylenoxy-, Butylenoxy-, Styroloxy-, Cyclohexyloxyeinheit oder eine durch Epoxidringöffnung aus einer Glycidylverbindung hervorgegangene Einheit ist,
- B: unabhängig von A eine gleiche oder unterschiedliche Ethylenoxy-, Propylenoxy-, Butylenoxy-, Styroloxy-, Cyclohexyloxyeinheit oder eine durch Epoxidringöffnung aus einer Glycidylverbindung hervorgegangene Einheit ist,
- m: gleich 0 bis 20,
- n: gleich 1 bis 40,
- x: eine ganze Zahl von 1 bis 6 ist
und die Monomereinheiten A und B in ihrer Abfolge beliebig sowohl blockartig als auch statistisch aneinandergereiht sein können.

Insbesondere können mit dem Verfahren Zusammensetzungen enthaltend Polyether der Formel (I) synthetisiert werden, die sich dadurch auszeichnen, dass sie hinsichtlich Strukturaufbau und Molmassenverteilung gezielt und reproduzierbar hergestellt werden können, nicht aufgearbeitet werden müssen und geruchlose kosmetische und/oder dermatologische Zubereitungen erlauben. Die kosmetischen und/oder dermatologischen Zubereitungen können 0,1 bis 80 Gew.-% der Polyether der Formel (I) enthalten. Die Menge variiert in Abhängigkeit von den weiteren verwendeten Zusatzstoffen und der gewünschten Härte oder Viskosität der Zielformulierung, beispielsweise einem Deo-Stift.

Der Stand der Technik referiert Alkoxylierungsverfahren, die sich der Katalyse mit Doppelmetallcyanid-Katalysatoren bedienen. Als Referenz sei hier z. B. auf EP-A-1017738, US 5,777,177, EP-A-0981407, WO 2006/002807 und EP-A-1474464 verwiesen.

Im Reaktionsgemisch liegt die Katalysatorkonzentration vorzugsweise bei > 0 bis 1.000 wppm (Massen-ppm), bevorzugt bei > 0 bis 500 wppm, besonders bevorzugt bei 0,1 bis 100 wppm und ganz besonders bevorzugt bei 1 bis 50 wppm. Diese Konzentration ist dabei bezogen auf die Gesamtmasse der gebildeten Polyetherpolyole; die Reaktionstemperatur beträgt etwa 60 bis 250 °C, bevorzugt von 90 bis 160 °C und besonders bevorzugt etwa 100 bis 130 °C. Der Druck, bei dem die Alkoxylierung stattfindet, beträgt vorzugsweise 0,02 bar bis 100 bar, bevorzugt 0,05 bis 20 bar absolut.

Als Alkohole R¹OH können in dem erfindungsgemäßen Verfahren insbesondere monofunktionelle Alkohole mit 4 bis 22 C-Atomen, bevorzugt 4 bis 18 C-Atomen wie Butanol, Myristylalkohol und Stearylalkohol, eingesetzt werden.

Als Epoxidmonomere im Sinne der Erfindung können neben Ethylenoxid, Propylenoxid, Butylenoxid, Styroloxid, 1,2-Dodecenoxid und Cyclohexenoxid alle bekannten weiteren mono- und polyfunktionellen Epoxidverbindungen inklusive der Glycidylether und -ester eingesetzt werden, und zwar einzeln als auch im Gemisch und sowohl statistisch als auch in blockartiger Abfolge.

Zum Starten der Reaktion kann es vorteilhaft sein, wenn zunächst ein Reaktionsgemisch, welches den DMC-Katalysator, gegebenenfalls in einem Suspendiermittel aufgeschlämmt, enthält, im Reaktor vorgelegt wird und diesem zumindest ein Alkylenoxid zudosiert wird. Das molare Verhältnis von Alkylenoxid zu reaktiven Gruppen, insbesondere OH-Gruppen im Startgemisch liegt vorzugsweise bei 0,1 bis 5 zu 1, bevorzugt bei 0,2 bis 2 zu 1. Es kann vorteilhaft sein, wenn vor der Zugabe des Alkylenoxids gegebenenfalls vorhandene, die Reaktion inhibierende Stoffe aus dem Reaktionsgemisch, z.B. durch Destillation, entfernt werden. Als Suspensionsmittel können entweder ein Polyether oder inerte Lösungsmittel benutzt werden oder vorteilhaft auch die Startverbindung, an die Alkylenoxid angelagert werden soll, oder ein Gemisch aus beiden.

Das Starten der Reaktion kann z. B. durch eine Drucküberwachung detektiert werden. Ein plötzlicher Abfall des Drucks im Reaktor zeigt bei gasförmigen Alkylenoxiden an, dass das Alkylenoxid eingebaut wird, die Reaktion somit gestartet und das Ende der Startphase erreicht ist.

Nach der Startphase, also nach Initialisierung der Reaktion, werden je nach angestrebter Molmasse entweder gleichzeitig Startverbindung und Alkylenoxid oder nur Alkylenoxid zudosiert. Alternativ kann auch ein beliebiges Gemisch von unterschiedlichen Alkylenoxiden addiert werden. Die Umsetzung kann z.B. zwecks Viskositätserniedrigung in einem inerten Lösemittel durchgeführt werden. Das molare Verhältnis der dosierten Alkylenoxide bezogen auf die eingesetzte Startverbindung, insbesondere bezogen auf die Anzahl der OH-Gruppen der eingesetzten Startverbindung, beträgt dabei vorzugsweise ausgedrückt als die Summe der Indices n+m gleich 1 bis 60. Vorzugsweise wird Propylenoxid immer nur im Gemisch mit einem weiteren Alkylenoxid eingesetzt.

Unter Startverbindungen werden im Rahmen der vorliegenden Erfindung Substanzen verstanden, die den Anfang (Start) des herzustellenden Polyethermoleküls bilden, das durch die Anlagerung von Alkylenoxid erhalten wird. Die in dem erfindungsgemäßen Verfahren eingesetzte Startverbindung ist vorzugsweise ausgewählt aus der Gruppe der Alkohole, Polyetherole, Phenole oder Carbonsäuren. Bevorzugt wird als Startverbindung ein ein- oder mehrwertiger Polyetheralkohol oder Alkohol R¹-OH eingesetzt.

Als OH-funktionelle Startverbindungen werden vorzugsweise Verbindungen mit Molmassen von 18 bis 1000 g/mol, insbesondere 100 bis 2000 g/mol und 1 bis 6, bevorzugt 1 bis 4 Hydroxylgruppen eingesetzt. Beispielhaft seien Butanol, Hexanol, Octanol, 2-Ethylhexanol, Nonanol, Isononanol, Decanol, Undecanol, Dodecanol, 2-Butyloctanol, Tridecanol, Tetradecanol, Hexadecylalkohol, 2-Hexyldecanol, Stearylalkohol, Isostearylalkohol oder Behenylalkohol genannt. Geeignet sind darüber hinaus Gemische der vorgenannten Alkohole.

Vorteilhaft werden niedermolekulare Polyetherole mit 1-6 Hydroxylgruppen und Molmassen von 100 bis 2000 g/mol, die ihrerseits zuvor durch DMC-katalysierte Alkoxylierung hergestellt wurden, als Starterverbindungen verwendet.

Neben Verbindungen mit aliphatischen und cycloaliphatischen OH-Gruppen eignen sich beliebige Verbindungen mit 1-20 phenolischen OH-Funktionen. Hierzu gehören beispielspweise Phenol, Alkyl- und Arylphenole, Bisphenol A und Novolake.

Als Reaktoren für die erfindungsgemäß beanspruchte Umsetzung können prinzipiell alle geeigneten Reaktortypen zum Einsatz kommen, die die Reaktion und ihre eventuell vorhandene Wärmetönung beherrschen lassen.

Die Reaktionsführung kann in verfahrenstechnisch bekannter Art und Weise kontinuierlich, semi-kontinuierlich oder auch batchweise erfolgen und lässt sich flexibel auf vorhandene produktionstechnische Einrichtungen abstimmen.

Neben herkömmlichen Rührkesselreaktoren können auch Strahlschlaufenreaktoren mit Gasphase und externen Wärmetauschern, wie beispielsweise in EP-A-0 419 419 beschrieben, oder internen Wärmetauscherrohren, wie in WO 01/62826 beschrieben, verwendet werden. Darüber hinaus können gasphasenfreie Loop-Reaktoren eingesetzt werden.

Bei der Dosierung der Edukte ist eine gute Verteilung der an der chemischen Umsetzung beteiligten Stoffe, d.h. der Alkylenoxide bzw. Glycidylverbindungen, Starter, DMC-Katalysator und gegebenenfalls Suspensionsmittel notwendig.

Nach der Alkylenoxid-Addition und eventueller Nachreaktion zur Vervollständigung des Alkylenoxidumsatzes kann das Produkt aufgearbeitet werden. Die hier erforderliche Aufarbeitung umfasst im Prinzip nur die Entfernung von nicht abreagiertem Alkylenoxid und eventuell weiteren, leicht flüchtigen Bestandteilen, üblicherweise durch Vakuumdestillation, Wasserdampf- oder Gasstrippen oder andere Methoden der Desodorierung. Die Entfernung leicht flüchtiger Nebenkomponenten kann sowohl batchweise als auch kontinuierlich erfolgen. Bei dem Verfahren auf Basis der DMC-Katalyse kann im Gegensatz zur konventionellen basen-katalysierten Alkoxylierung im Normalfall auf eine Filtration verzichtet werden.

Es ist bei Bedarf möglich, den DMC-Katalysator aus dem fertigen Polyetheralkohol zu entfernen. Für die meisten Einsatzgebiete kann er jedoch im Polyetheralkohol verbleiben. Prinzipiell möglich, wenn auch nicht bevorzugt, ist es, den DMC-Katalysator abzutrennen und wieder zu verwenden, wie beispielsweise in WO 01/38421 beschrieben. Diese Verfahrensweise ist jedoch für die großtechnische Herstellung von Polyetheralkoholen meist zu aufwändig.

Es ist üblich, den gebildeten Polyetheralkohol gegen den thermooxidativen Abbau zu stabilisieren. Dies erfolgt üblicherweise durch den Zusatz von Stabilisatoren, zumeist sterisch gehinderten Phenolen wie z.B. BHT - Butylhydroxytoluen, BHA - Butylhydroxyanisol, TBHQ - tert-Butylhydrochinon oder Pentaerythrittetrakis(3-(3,5-di-tert-butyl-4-hydroxyphenyl)propionat).

Die Anlagerung der Alkylenoxid- bzw. allgemeiner gefasst Epoxidverbindungen geschieht vorzugsweise bei einer Temperatur von 60 bis 250 °C, bevorzugt von 90 bis 160 °C und besonders bevorzugt bei einer Temperatur von 100 bis 130 °C. Der Druck, bei dem die Alkoxylierung stattfindet, beträgt vorzugsweise 0,02 bar bis 100 bar, bevorzugt 0,05 bis 20 bar absolut. Durch die Durchführung der Alkoxylierung bei Unterdruck kann die Reaktion sehr sicher durchgeführt werden. Gegebenenfalls kann die Alkoxylierung in Gegenwart eines Inertgases (z.B. Stickstoff) und auch bei Überdruck durchgeführt werden.

Die Verfahrensschritte können bei identischen oder verschiedenen Temperaturen ausgeführt werden. Die im Reaktor zum Reaktionsstart vorgelegte Mischung aus Startsubstanz, und DMC-Katalysator kann vor Beginn der Dosierung der Alkylenoxide gemäß der Lehre von WO 98/52689 durch Strippen vorbehandelt werden. Dabei wird über die Reaktorzuführung ein Inertgas dem Reaktionsgemisch zugemischt und mit Hilfe einer an das Reaktorsystem angeschlossenen Vakuumanlage werden leichter flüchtige Komponenten durch Anlegen eines Unterdrucks aus dem Reaktionsgemisch entfernt. Auf diese einfache Weise können aus dem Reaktionsgemisch Stoffe, die den Katalysator inhibieren können, wie z. B. niedere Alkohole oder Wasser, entfernt werden. Die Zugabe von Inertgas und das gleichzeitige Entfernen der leichter flüchtigen Komponenten kann insbesondere beim Anfahren/Starten der Reaktion von Vorteil sein, da durch die Zugabe der Reaktanden oder durch Nebenreaktionen auch inhibierende Verbindungen in das Reaktionsgemisch gelangen können.

Als DMC-Katalysator können alle bekannten DMC-Katalysatoren, vorzugsweise solche, die Zink und Kobalt aufweisen, bevorzugt solche, die Zinkhexacyanocobaltat(III) aufweisen, eingesetzt werden. Vorzugsweise werden die in US 5,158,922, US 20030119663, WO 01/80994 oder in den oben genannten Schriften beschriebenen DMC-Katalysatoren eingesetzt. Die Katalysatoren können amorph oder kristallin sein.

Im Reaktionsgemisch liegt die Katalysatorkonzentration vorzugsweise bei > 0 bis 1.000 wppm (Massen-ppm), bevorzugt bei > 0 bis 500 wppm, besonders bevorzugt bei 0,1 bis 100 wppm und ganz besonders bevorzugt bei 1 bis 50 wppm. Diese Konzentration ist dabei bezogen auf die Gesamtmasse der Polyetherpolyole.

Vorzugsweise wird der Katalysator nur einmal in den Reaktor dosiert. Die Katalysatormenge ist so einzustellen, dass eine ausreichende katalytische Aktivität für das Verfahren gegeben ist. Der Katalysator kann als Feststoff oder in Form einer Katalysatorsuspension dosiert werden. Wird eine Suspension verwendet, so eignet sich insbesondere der Startpolyether als Suspensionsmittel. Bevorzugt wird aber auf eine Suspendierung verzichtet.

Die kosmetischen Zubereitungen können z. B. mindestens eine zusätzliche Komponente enthalten, ausgewählt aus der Gruppe der
Emollients,
Emulgatoren und Tenside,
Verdicker/Viskositätsregler/Stabilisatoren,
UV-Lichtschutzfilter,
Antioxidantien und Vitamine,
Hydrotrope (oder Polyole),
Fest- und Füllstoffe,
Filmbildner,
Perlglanzadditive,
Deodorant- und Antitranspirantwirkstoffe,
Esteraseinhibitoren,
Insektrepellentien,
Selbstbräuner,
Konservierungsstoffe,
Konditioniermittel,
Parfüme,
Farbstoffe,
Biogene Wirkstoffe,
Pflegeadditive,
Überfettungsmittel,
Lösungsmittel.

Als Emollients können alle kosmetischen Öle insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen eingesetzt werden. Ebenso sind die Veresterungsprodukte aliphatischer, difunktioneller Alkohole mit 2 bis 36 C-Atomen mit monofunktionellen aliphatischen Carbonsäuren mit 1 bis 22 C-Atomen einsetzbar. Des Weiteren eignen sich langkettige Arylsäureester wie z.B. Ester der Benzoesäure, z.B. Benzoesäureester von linearen oder verzweigten, gesättigten oder ungesättigten Alkoholen mit 1 bis 22 C-Atomen, oder auch Benzoesäureisostearylester oder Benzoesäureoctyldocecylester. Weitere als Emollients und Ölkomponenten geeignete Monoester sind z.B. die Methylester und Isopropylester von Fettsäuren mit 12 bis 22 C-Atomen wie z.B. Methyllaurat, Methylstearat, Methyloleat, Methylerucat, Isopropylpalmitat, Isopropylmyristat, Isopropylstearat, Isopropyloleat. Andere geeignete Monoester sind z.B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z.B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z.B. im Jojobaöl oder im Spermöl vorliegen. Geeignete Dicarbonsäureester sind z.B. Di-n-butyladipat, Di-n-butyl-sebacat, Di-(2-ethylhexyl)-adipat, Di-(2-hexyldecyl)-succinat, Di-isotridecylacelaat. Geeignete Diolester sind z.B. Ethylenglycoldioleat, Ethylenglycol-di-isotridecanoat, Propylenglycol-di-(2-ethylhexanoat), Butandiol-diisostearat, Butandiol-di-caprylat/caprat und Neopentylglycoldicaprylat. Weitere Fettsäureester, die als Emollients eingesetzt werden können, sind z.B. C₁₂₋₁₅ Alkylbenzoat, Dicaprylylcarbonat, Diethylhexylcarbonat. Ebenso als Emollients und Ölkomponente können längerkettige Triglyceride, d.h. dreifache Ester des Glycerins mit drei Säuremolekülen, wovon mindestens eine längerkettig ist, eingesetzt werden. Hier seien beispielhaft Fettsäuretriglyceride erwähnt; als solche können beispielsweise natürliche, pflanzliche Öle, z.B. Olivenöl, Sonnenblumenöl, Sojaöl, Erdnußöl, Rapsöl, Mandelöl, Sesamöl, Avocadoöl, Rizinusöl, Kakaobutter, Palmöl aber auch die flüssigen Anteile des Kokosöls oder des Palmkernöls sowie tierische Öle wie z.B. Haifischlebertran, Dorschleberöl, Walöl, Rindertalg und Butterfett, Wachse wie Bienenwachs, Karnaubapalmwachs, Spermazet, Lanolin und Klauenöl, die flüssigen Anteile des Rindertalgs oder auch synthetische Triglyceride von Capryl-Caprinsäure-Gemischen, Triglyceride aus technischer Ölsäure, Triglyceride mit Isostearinsäure, oder aus Palmitinsäure-Ölsäure-Gemischen als Emollients und Ölkomponenten eingesetzt werden. Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan, Ceresin. Weiterhin sind auch lineare oder verzweigte Fettalkohole wie Oleylalkohol oder Octyldodecanol, sowie Fettalkoholether wie Dicaprylyl Ether einsetzbar. Geeignete Siliconöle und -wachse sind z.B. Polydimethylsiloxane, Cyclomethylsiloxane, sowie aryl- oder alkyl- oder alkoxy- substituierte Polymethylsiloxane oder Cyclomethylsiloxane. Als weitere Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-Cₗ₃-Carbonsäuren mit linearen C₆-C₂₂-Fettalkoholen, Ester von linearen C₆-C₂₂-Fettsäuren mit verzweigten C₈-C₁₈-Alkoholen, insbesondere 2-Ethylhexanol oder Isononanol, Ester von verzweigten C₆-C₁₃-Carbonsäuren mit verzweigten Alkoholen, insbesondere 2-Ethylhexanol oder Isononanol, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen, Dialkylether, Ringöffnungsprodukte von epoxidierten Fettsäureestern mit Polyolen, Siliconöle und/oder aliphatische bzw. naphthenische Kohlenwasserstoffe in Betracht.

Die Ölkörper/Emollients (erfindungsgemäße Polyetherpolyole plus weitere Ölkörper) sind üblicherweise in einer Gesamtmenge von 0,1-90 Gew.-%, insbesondere 0,1-80 Gew.-%, insbesondere 0,5 bis 70 Gew.-%, bevorzugt 1 bis 60 Gew.-%, insbesondere 1 bis 40 Gew.-% und vorzugsweise 5 bis 25 Gew.-% enthalten. Die weiteren Ölkörper sind üblicherweise in einer Menge von 0,1 bis 40 Gew.-% bezogen auf das Gesamtgewicht der Zubereitung enthalten.

Als Emulgatoren oder Tenside können nichtionische, anionische, kationische oder amphotere Tenside eingesetzt werden.

Als nichtionogene Emulgatoren oder Tenside können Verbindungen aus mindestens einer der folgenden Gruppen eingesetzt werden: Anlagerungsprodukte von 2 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C_{12/18}-Fettsäuremono- und -diester von Anlagerungsprodukten von 1 bis 100 Mol Ethylenoxid an Glycerin, Glycerinmono- und -diester und Sorbitanmono- und -diester von gesättigten und ungesättigten Fettsäuren mit 6 bis 22 Kohlenstoffatomen und deren Ethylenoxidanlagerungsprodukte, Alkylmono- und -oligoglycoside mit 8 bis 22 Kohlenstoffatomen im Alkylrest und deren Ethylenoxidanlagerungsprodukte, Anlagerungsprodukte von 2 bis 200 Mol Ethylenoxid an Ricinusöl und/oder gehärtetes Ricinusöl,
Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose),
Mono-, Di- und Trialkylphosphate sowie Mono-, Di- und/oder Tri-PEG-alkylphosphate und deren Salze,
Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 4/12 oder 20/20 oder 18/18 oder 17/18 oder 15/15,
Polysiloxan-Polyalkyl-Polyether-Copolymere bzw. entsprechende Derivate, wie z.B. Lauryl oder Cetyl Dimethicone Copolyole, insbesondere Cetyl PEG/PPG-10/1 Dimethicone (ABIL^{®} EM 90 (Evonik Goldschmidt GmbH)),
Mischester aus Pentaerythrit, Fettsäuren, Citronensäure und Fettalkohol gemäß DE 11 65 574 und/oder Mischester von Fettsäuren mit 6 bis 22 Kohlenstoffatomen, Methylglucose und Polyolen, wie z.B. Glycerin oder Polyglycerin, Zitronensäureester wie z.B. Glyceryl Stearate Citrate, Glyceryl Oleate Citrate und Dilauryl Citrate.

Anionische Emulgatoren oder Tenside können wasserlöslich machende anionische Gruppen wie z.B. eine Carboxylat-, Sulfat-, Sulfonat- oder Phosphat-Gruppe und einen lipophilen Rest enthalten. Hautverträgliche anionische Tenside sind dem Fachmann in großer Zahl bekannt und im Handel erhältlich. Dabei kann es sich um Alkylsulfate oder Alkylphosphate in Form ihrer Alkali, Ammonium- oder Alkanolammoniumsalze, Alkylethersulfate, Alkylethercarboxylate, Acylsarkosinate sowie Sulfosuccinate und Acylglutamate in Form ihrer Alkali- oder Ammoniumsalze handeln.

Auch kationische Emulgatoren und Tenside können zugesetzt werden. Als solche können insbesondere quaternäre Ammoniumverbindungen, insbesondere solche, versehen mit mindestens einer linearen und/oder verzweigten, gesättigten oder ungesättigten Alkylkette mit 8 bis 22 C-Atomen, eingesetzt werden, so etwa Alkyltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid.

Weiterhin können Monoalkylamidoquats wie z.B. Palmitamidopropyltrimethylammoniumchlorid oder entsprechende Dialkylamidoquats eingesetzt werden.

Weiterhin können biologisch gut abbaubare quaternäre Esterverbindungen eingesetzt werden, bei denen es sich um quaternierte Fettsäureester auf Basis von Mono-, Di- oder Triethanolamin handeln kann. Weiterhin können Alkylguanidiniumsalze als kationische Emulgatoren beigesetzt sein.

Typische Beispiele für milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere beispielsweise auf Basis von Weizenproteinen.

Weiterhin ist es möglich, amphotere Tenside wie z.B. Betaine, Amphoacetate oder Amphopropionate einzusetzen, so z.B. Substanzen wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

Von den ampholytischen Tensiden können solche oberflächenaktiven Verbindungen eingesetzt werden, die außer einer C_{8/18}-Alkyl- oder -Acylgruppe im Molekül mindestens eine freie Aminogruppe und mindestens eine -COOH- oder -SO₃H- Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Weitere Beispiele ampholytischer Tenside sind das N-Kokosalkylaminopropionat, das Kokosacylaminoethylaminopropionat und das C_{12/18}-Acylsarcosin. Die erfindungsgemäßen Zubereitungen enthalten den/die Emulgator(en) bzw. Tenside üblicherweise in einer Menge von 0 bis 40 Gew.-%, vorzugsweise 0,1 bis 20 Gew.-%, bevorzugt 0,1 bis 15 Gew.-% und insbesondere 0,1 bis 10 Gew.-% bezogen auf das Gesamtgewicht der Zubereitung.

Geeignete Verdicker sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z. B. Carbopole TM oder Synthalene TM), Polyacrylamide, Polyvinylalkohol und Polyvinylpyrrolidon, Tenside wie beispielsweise ethoxylierte Fettsäure-glyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als Verdicker zur Verdickung von Ölphasen kommen alle dem Fachmann bekannten Verdickungsmittel in Frage. Insbesondere sind dabei zu nennen Wachse, wie hydriertes Castorwachs, Bienenwachs oder Microwachs. Weiterhin können auch anorganische Verdickungsmittel eingesetzt werden wie Silica, Alumina oder Schichtsilikate (z.B. Hectorit, Laponit, Saponit). Diese anorganischen Ölphasenverdicker können dabei hydrophob modifiziert sein. Zur Verdickung/Stabilisierung von Wasser-in-Öl-Emulsionen können dabei insbesondere Aerosile, Schichtsilikate und/oder Metallsalze von Fettsäuren, wie z.B. Magnesium-, Aluminium- und/oder Zinkstearat bzw. -ricinoleat eingesetzt werden.

Als Viskositätsregler für wässrige Tensidsysteme können z.B. NaCl, niedermolekulare nichtionische Tenside, wie Cocoamide DEA/MEA und Laureth-3, oder polymere, hochmolekulare, assoziative, hochethoxylierte Fettderivate, wie PEG-200 Hydrogenated Glyceryl Palmate enthalten sein.

Als UV-Lichtschutzfilter können beispielsweise organische Substanzen eingesetzt werden, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche UVB-Lichtschutzfilter sind z.B. zu nennen:
3-Benzylidencampher und dessen Derivate, z.B. 3-(4-Methylbenzyliden)campher, 4-Aminobenzoesäurederivate, wie z.B. 4-(Dimethylamino)benzoesäure-2-ethylhexylester, 4-(Dimethylamino)benzoesäure-2-ethylhexylester und 4-(Dimethylamino)-benzoesäureamylester, Ester der Zimtsäure, wie z.B. 4-Methoxyzimtsäure-2-ethylhexylester, 4-Methoxyzimtsäureisopentylester, 2-Cyan-3-phenyl-zimtsäure-2-ethylhexylester (Octocrylene), Ester der Salicylsäure, wie z.B. Salicylsäure-2-ethylhexylester, Salicylsäure-4-isopropylbenzylester, Salicylsäurehomomenthylester,
Derivate des Benzophenons, wie z.B. 2-Hydroxy-4-methoxybenzophenon, 2-Hydroxy-4-methoxy-4'-methylbenzophenon, 2,2'-Dihydroxy-4-methoxybenzophenon,
Ester der Benzalmalonsäure, wie z.B. 4-Methoxybenzmalonsäuredi-2-ethylhexyester,
Triazinderivate, wie z.B. 2,4,6-Trianilino-(p-carbo-2'-ethyl-1'-hexyloxy)-1,3,5-triazin, Octyltriazon und solche in der EP 1180359 und DE 2004/027475 beschriebenen,
Propan-1,3-dione, wie z.B. 1-(4-tert.Butylphenyl)-3-(4'-methoxyphenyl)propan-1,3-dion.

Als wasserlösliche UVB-Lichtschutzfilter kommen in Frage: 2-Phenylbenzimidazol-5-sulfonsäure und deren Alkali-, Erdalkali-, Ammonium-, Alkylammonium-, Alkanolammonium- und Glucammoniumsalze,
Sulfonsäurederivate von Benzophenon, wie z.B. 2-Hydroxy-4-methoxybenzophenon-5-sulfonsäure und ihre Salze, Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UVA-Lichtschutzfilter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden.

Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z.B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazole-2-yl)-4-(1, 1, 3, 3-tetramethylbutyl)-phenol} mit einer Partikelgröße von < 200 nm, das z.B. als 50 %ige wässrige Dispersion erhältlich ist.

Weitere geeignete UV-Lichtschutzfilter sind der Übersicht von P. Finkel in SÖFW-Journal 122, 543 (1996) zu entnehmen.

Die erfindungsgemäßen Zubereitungen können die UV-Lichtschutzfilter in Mengen von 0 bis 30 Gew.-%, vorzugsweise 0 bis 20 Gew.-% bezogen auf das Gesamtgewicht der Zubereitung enthalten.

Neben den beiden vorgenannten Gruppen primärer UV-Lichtschutzfilter können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Als Antioxidantien und Vitamine können z.B. Superoxid-Dismutase, Tocopherol (Vitamin E), Tocopherolsorbat, Tocopherolacetat, andere Ester von Tocopherol, Dibutylhydroxytoluol und Ascorbinsäure (Vitamin C) und ihre Salze sowie deren Derivate (z.B. Magnesium Ascorbylphosphat, Natrium Ascorbylphosphat, Ascorbylsorbat), Ascorbyl Ester von Fettsäuren, butylierte Hydroxybenzoesäure und ihre Salze, Peroxide wie z.B. Wasserstoffperoxid, Perborate, Thioglycolate, Persulfatsalze, 6-Hydroxy-2, 5, 7, 8-Tetramethylchroman-2-Carboxylsäure (TROLOX.RTM), Gallussäure und ihre Alkylester, Harnsäure und ihre Salze und Alkylester, Sorbinsäure und ihre Salze, Liponsäure, Ferulasäure, Amine (z.B. N,N-Diethylhydroxylamin, Amino-Guanidine), Sulfhydryl-Verbindungen (z.B. Glutathion), Dihydroxy-Fumarsäure und ihre Salze, Glycinpidolat, Argininpilolat, Nordihydroguaiaretissche Säure, Bioflavonoide, Curcumin, Lysin, L-Methionin, Prolin, Superoxid Dismutase, Silymarin, Tee Extract, Grapefruit Schalen/Kern Extrakt, Melanin, Rosmarin Extrakt, Thioctansäure, Resveratrol, Oxyresveratrol, etc. eingesetzt werden.

Als Hydrotrope können zur Verbesserung des Fließverhaltens und der Anwendungseigenschaften beispielsweise Ethanol, Isopropylalkohol oder Polyole eingesetzt werden. Polyole, die hier in Betracht kommen, können 2 bis 15 Kohlenstoffatome und mindestens zwei Hydroxylgruppen besitzen. Typische Beispiele sind:
Glycerin Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton, technische Oligoglyceringemische mit einem Eigenkondensationsgrad von 1,5 bis 10 wie etwa technische Diglyceringemische mit einem Diglyceringehalt von 40 bis 50 Gew.-%,
Methylolverbindungen, wie insbesondere Trimethylolethan, Trimethylolpropan, Trimethylolbutan, Pentaerythrit und Dipentaerythrit, Niedrigalkylgucoside, insbesondere solche mit 1 bis 4 Kohlenstoffatomen im Alkylrest, wie beispielsweise Methyl- und Butylglucosid, Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose, Aminozucker, wie beispielsweise Glucamin.

Als Feststoffe können beispielsweise Eisenoxidpigmente, Titandioxid oder Zinkoxidpartikel und die zusätzlich unter "UV-Schutzmittel" genannten eingesetzt werden. Weiterhin können auch Partikel eingesetzt werden, die zu speziellen sensorischen Effekten führen, wie etwa Nylon-12, Bornitrid, Polymerpartikel wie etwa Polyacrylat- oder Polymethylacrylatpartikel oder Siliconelastomere. Einsetzbare Füllstoffe umfassen Stärke und Stärkederivate, wie Tapiocastärke, Distärkephosphat, Aluminium- bzw. Natrium-Stärke, Octenylsuccinat sowie Pigmente, die weder hauptsächlich UV-Filter- noch färbende Wirkung haben, beispielsweise Aerosile® (CAS-Nr. 7631-86-9).

Die Feststoffe können vorteilhaft im Sinne der vorliegenden Erfindung auch in Form kommerziell erhältlicher öliger oder wässriger Vordispersionen zur Anwendung kommen. Die erfindungsgemäßen Zubereitungen enthalten üblicherweise 0 bis 40 Gew.-% Pigmente bezogen auf das Gesamtgewicht der Zubereitung.

Als Filmbildner zur z.B. Verbesserung der Wasserfestigkeit können beispielsweise eingesetzt werden: Polyurethane, Dimethicone, Copolyol, Polyacrylate oder PVP/VA Copolymer (PVP = Polyvinylpyrrolidon, VA = Vinylacetat). Als fettlösliche Filmbildner können eingesetzt werden: z.B. Polymere auf Basis von Polyvinylpyrrolidon (PVP), Copolymere des Polyvinylpyrrolidons, PVP/Hexadecen-Copolymer oder das PVP/Eicosen-Copolymer.

Als Perlglanzadditive können z.B. Glycoldistearate oder PEG-3 Distearat eingesetzt werden.

Als Deodorantwirkstoffe kommen z.B. Geruchsüberdecker wie die gängigen Parfümbestandteile, Geruchsabsorber, beispielsweise die in der Patentoffenlegungsschrift DE 40 09 347 beschriebenen Schichtsilikate, von diesen insbesondere Montmorillonit, Kaolinit, Illit, Beidelit, Nontronit, Saponit, Hectorit, Bentonit, Smectit, ferner beispielsweise Zinksalze der Ricinolsäure oder Talkum in Frage. Keimhemmende Mittel sind ebenfalls geeignet, eingearbeitet zu werden. Keimhemmende Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbonilid, quaternäre Ammoniumverbindungen, Nelkenöl, Minzöl, Thymianöl, Triethylcitrat, Farnesol (3,7,11-Trimethyl-2,6,10-dodecatrien-1-ol), Ethylhexyl glycerylether, Polyglyceryl-3 caprylat (TEGO^{®} Cosmo P813, Evonik Goldschmidt GmbH), sowie die in den Patentoffenlegungsschriften DE 198 55 934, DE 37 40 186, DE 39 38 140, DE 42 04 321, DE 42 29 707, DE 42 29 737, DE 42 38 081, DE 43 09 372, DE 43 24 219 und EP 666 732 beschriebenen wirksamen Agenzien.

Die erfindungsgemäßen Zubereitungen können die Deodorantwirkstoffe in Mengen von 0,1 bis 30 Gew.-%, vorzugsweise 1 bis 20 Gew.-% und insbesondere 2 bis 10 Gew.-% bezogen auf das Gesamtgewicht der Zubereitung enthalten.

Bei Antitranspirantwirkstoffen handelt es sich um Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit 1,2-Propylenglycol, Aluminiumhydroxyallantoinat, Aluminiumchloridtartrat, Aluminium-Zirkonium-Trichlorohydrat, Aluminium-Zirkoniumtetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt.

Die erfindungsgemäßen Zubereitungen können die Antitranspirantwirkstoffe in Mengen von 1 bis 50 Gew.-%, vorzugsweise 5 bis 30 Gew.-% und insbesondere 8 bis 25 Gew.-% bezogen auf das Gesamtgewicht der Zubereitung enthalten.

Esteraseinhibitoren:
Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen.
Als Esteraseinhibitoren geeignet sind vorzugsweise Trialkylcitrate wie Tri-methylcitrat, Tripropylcitrat, Triisopropylcitrat, Tributylcitrat und insbesondere Triethylcitrat (Hydagen® CAT, Cognis GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-,
   Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Zubereitungen können die Esteraseinhibitoren in Mengen von 0,01 bis 20 Gew.-%, vorzugsweise 0,1 bis 10 Gew.-% und insbesondere 0,3 bis 5 Gew.-% bezogen auf das Gesamtgewicht der Zubereitung enthalten.

Als Insekten-Repellentien können beispielsweise N,N-Diethyl-m-toluamid, 1,2-Pentandiol oder Insect Repellent 3535 eingesetzt werden.

Als Selbstbräuner können z.B. Dihydroxyaceton und Erythrulose eingesetzt werden.

Als Konservierungsstoffe eignen sich beispielsweise Phenoxyethanol, Formaldehydlösung, Parabene, Pentandiol oder Sorbinsäure sowie die unter der Bezeichnung Surfacine® bekannte Silberkomplexe. Weiterhin eignen sich Konservierungsmittel die in WO 07/048757 beschriebenen 1,2-Alkandiole mit 5 bis 8 C-Atomen.

Als Konservierungsmittel eignen sich insbesondere die gemäß Annex VI der EU Direktive 76/768/EEC (in der aktuellen Fassung) zugelassenen Stoffe, auf die hier explizit Bezug genommen wird.

Als Konditioniermittel können z.B. organische quaternäre Verbindungen wie Cetrimoniumchlorid, Dicetyldimoniumchlorid, Behentrimoniumchlorid, Distearyldimoniumchlorid, Behentrimoniummethosulfat, Distearoylethyldimoniumchlorid, Palmitamidopropyltrimoniumchlorid, Guar Hydroxypropyltrimoniumchlorid, Hydroxypropylguar Hydroxypropyltrimoniumchlorid, oder Quaternium-80 oder auch Aminderivate wie z.B. Aminopropyldimethicone oder Stearamidopropyldimethylamine verwendet werden.

Als Parfüme können natürliche oder synthetische Riechstoffe oder Gemische daraus eingesetzt werden. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexyl-acetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen z.B. die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Es können Mischungen verschiedener Riechstoffe eingesetzt werden, die gemeinsam eine ansprechende Duftnote erzeugen. Auch ätherische Öle geringer Flüchtigkeit, die meist als Aromakomponenten eingesetzt werden, eignen sich als Parfüme, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Es können Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt werden.

Die erfindungsgemäßen Zubereitungen können die Parfüme bzw. Parfümgemische in Mengen von 0 bis 2 Gew.-%, vorzugsweise 0,01 bis 1,5 Gew.-% und insbesondere 0,05 bis 1 Gew.-% bezogen auf das Gesamtgewicht der Zubereitung enthalten.

Als Farbstoffe können die für kosmetische Zwecke geeigneten und zugelassenen Substanzen eingesetzt werden, wie sie beispielsweise in der Publikation "Kosmetische Färbemittel" der Farbstoffkommission der Deutschen Forschungsgemeinschaft, Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung, eingesetzt.

Unter biogenen Wirkstoffen sind beispielsweise Tocopherol, Tocopherolacetat, Tocopherolpalmitat, Ascorbinsäure, Polyphenole, Desoxyribonucleinsäure, Coenzym Q10, Retinol, AHA-Säuren, Aminosäuren, Hyaluronsäure, alpha-Hydroxysäuren, Isoflavone, Polyglutaminsäure, Creatin (und Creatinderivate), Guanidin (und Guanidinderivate), Pseudoceramide, essentielle Öle, Peptide, Proteinhydrolysate, Pflanzenextrakte, Bisabolol, Allantoin, Panthenol, Phytantriol, Idebenon, Lakritz Extrakt, Glycyrrhizidin und Idebenon, Skleroglucan, β-Glucan, Santalbinsäure und Vitaminkomplexe zu verstehen. Beispiele für Pflanzenextrakte sind Kastanien Extrakt, Kamillen Extrakt, Rosmarin Extrakt, schwarze und rote Johannisbeer Extrakt, Birken Extrakt, Hagebutten Extrakt, Algen Extrakte, Grüner Tee Extrakt, Aloe Extrakt, Ginseng Extrakt, Ginko Extrakt, Grapefruit Extrakt, Calendula Extrakt, Kampher, Thymus Extrakt, Mangosteen Extrakt, Cystus Extrakt, Terminalia Arjuna Extrakt, Hafer Extrakt, Oregano Extrakt, Himbeer Extrakt, Erdbeer Extrakt, etc.

Zu den biogenen Wirkstoffen können auch die sogenannten Barriere Lipids gezählt werden, für welche beispielhaft Ceramide, Phytosphingosin und Derivate, Sphingosin und Derivate, Sphinganin und Derivate, Pseudoceramide, Phospholipide, Lysophospholipide, Cholesterin und Derivate, Cholesteryl Ester, freie Fettsäuren, Lanolin und Derivate, Squalan, Squalen und verwandte Substanzen genannt werden.
Zu den biogenen Wirkstoffen werden im Sinne der Erfindung auch anti-Akne wie z.B. Benzylperoxid, Phytosphingosin und Derivate, Niacinamid Hydroxybenzoat, Nicotinaldehyd, Retinolsäure und Derivate, Salicylsäure und Derivate, Citronellsäure etc. und anti-Cellulite wie z.B. Xanthin Verbindungen wie Coffein, Theophyllin, Theobromin und Aminophyllin, Carnitin, Carnosin, Salicyloyl Phytosphingosin, Phytosphingosine, Santalbinsäure etc. gezählt, ebenso wie Antischuppenmittel wie beispielsweise Salicylsäure und Derivate, Zink Pyrithion, Selensulfid, Schwefel, Ciclopiroxolamin, Bifonazol, Climbazol, Octopirox und Actirox etc, ebenso wie Adstringetien wie z.B. Alkohol, Aluminium Derivate, Gallsäure, Pyridoxinsalicylat, Zinksalze wie z.B. Zinksulfat, -acetat, -chlorid, -lactat, Zirconiumchlorohydrate etc. Ebenso können Bleichmittel wie Kojisäure, Arbutin, Vitamin C und Derivate, Hydroquinon, Turmeric oil, Creatinin, Sphingolipide, Niacinamid, etc. zu den biogenen Wirkstoffen gezählt werden.

Als Pflegeadditive können z.B. ethoxylierte Glycerin-Fettsäureester, wie beispielweise PEG-7 Glycerin Cocoate, oder kationische Polymere, wie beispielsweise Polyquaternium-7 oder Polyglycerinester enthalten sein.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Lösungsmittel können z.B. aliphatische Alkohole wie Ethanol, Propanol oder 1,3-Propandiol, cyclische Carbonate wie Ethylencarbonat, Propylencarbonat, Glycerincarbonat, Ester von Mono- oder Polycarbonsäuren wie Ethylacetat, Ethyllactat, Dimethyladipat und Diethyladipat, Propylenglycol, Dipropylenglycol, Glycerin, Glycerincarbonat oder Wasser eingesetzt werden.

Ein weiterer Gegenstand der Erfindung sind dickflüssige bis halbfeste und bis hin zu cremefeste kosmetische und/oder dermatologische Zubereitungen, die in einem für kosmetische Stifte geeigneten oder Deoroller-Behältnis oder einem Zerstäuberpumpen-Behältnis an den Verbraucher weitergegeben werden können.

Ein weiterer Gegenstand der Erfindung sind kosmetische und/oder dermatologische Zubereitungen, die neben anderen kosmetischen Zusatzstoffen Parfüm und/oder Duftstoffe enthalten.

Ein weiterer Gegenstand der Erfindung ist die Verwendung einer Antitranspirantformulierung basierend auf den Zubereitungen zur Auftragung auf die menschliche Haut, insbesondere zur Verminderung der Schweissbildung.

Weitere Gegenstände der Erfindung ergeben sich aus den Ansprüchen, deren Offenbarungsgehalt vollumfänglich Teil dieser Beschreibung ist.

Die erfindungsgemäßen kosmetischen Zubereitungen und deren Zubereitung und Verwendung werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören.

### Beispiele:

### 1) Herstellbeispiele:

### 1a) PPG Butyl Ether mittels DMC-Katalyse (erfindungsgemäß)

In einem 3 Liter Autoklaven werden 400 g Polypropylenglykolmonobutylether (massenmittlere Molmasse M_{w} = 400 g/mol) und 0,03 g Zinkhexacyanocobaltat-DMC-Katalysator unter Stickstoff vorgelegt, dann auf 130 °C aufgeheizt. Der Reaktor wird bis auf einen Innendruck von 30 mbar evakuiert, um evtl. vorhandene flüchtige Inhaltsstoffe destillativ zu entfernen. Zur Aktivierung des DMC-Katalysators wird eine Portion von 20 g Propylenoxid zugeführt. Nach Anspringen der Reaktion und erfolgtem Innendruckabfall werden in 80 min gleichzeitig weitere 550 g Propylenoxid und 74 g n-Butanol kontinuierlich unter Kühlen zugespeist. Abschließend werden nochmals 956 g Propylenoxid innerhalb von 45 min bei 130 °C und max. 1,5 bar Reaktorinnendruck zudosiert. An die 30-minütige Nachreaktion bei 130 °C schließt sich die Entgasungsstufe an. Dabei werden flüchtige Anteile wie restliches Propylenoxid im Vakuum bei 130 °C abdestilliert. Der fertige farblose Polyether wird auf unter 90 °C abgekühlt und aus dem Reaktor abgelassen. Er besitzt eine OH-Zahl von 55 mg KOH/g, eine Säurezahl von < 0,1 mg KOH/g und lt. GPC eine mittlere Molmasse M_{w} von 1100 g/mol bzw. ein Mₙ von 1036 g/mol sowie eine Polydispersität M_{w}/Mₙ von 1,06, gemessen gegen Polypropylenglykol-Standard.

### 1b) PPG Butyl Ether mittels KOH-Katalyse (nicht erfindungsgemäß)

In einem 3 Liter Autoklaven werden 148 g n-Butanol und 5,6 g Kaliumhydroxid unter Stickstoff vorgelegt und auf 130 °C aufgeheizt. 2050 g Propylenoxid werden unter Kühlen in ca. 7 h so zugespeist, dass bei 130 °C der Reaktorinnendruck 2,5 bar nicht übersteigt. An die 3-stündige Nachreaktion bei 130 °C schließt sich die Entgasungsstufe an, um flüchtige Anteile wie restliches Propylenoxid im Vakuum bei 130 °C abzudestillieren. Anschließend wird bei 130 °C eine weitere Stunde mit Stickstoff gestrippt. Der noch alkalische Polyether wird auf 70 °C abgekühlt und mit wässeriger Schwefelsäure auf einen pH-Wert von 7 eingestellt. Anschließend wird das Produkt bei 100-110 °C und max. 50 mbar mit Wasserdampf und Stickstoff gestrippt und die Salzreste durch eine Filtration bei ca. 60 °C abgetrennt.
Der fertige Polyether hat eine OH-Zahl von 53 mg KOH/g, eine Säurezahl von < 0,1 mg KOH/g und lt. GPC eine mittlere Molmasse M_{w} von 1208 g/mol bzw. ein Mₙ von 1093 g/mol sowie eine Polydispersität M_{w}/Mₙ von 1,11, gemessen gegen Polypropylenglykol-Standard.

### 2) Rezepturbeispiele:

AP/Deo Stick Formulierungen
Alle Angaben beziehen sich auf Gewichtsprozent (Gew.-%) sofern es nicht anderweitig angegeben ist.

| **Beispiel** | **2A** | **2B** |
|---|---|---|
| Stearyl Alkohol | 18 | 18 |
| Hydrogenated Castor Oil | 4 | 4 |
| C₁₂-₁₅ Alkyl Benzoate | 6 | 6 |
| PPG-14 Butyl Ether (gem. Herstellbeispiel 1a) | 8 | |
| PPG-14 Butyl Ether (gem. Herstellbeispiel 1b) | | 8 |
| Cyclomethicone | 42 | 42 |
| Talc | 2 | 2 |
| Aluminum Zirconium Tetrachlorohydrex GLY | 20 | 20 |

### 3) Geruchsbewertung:

Die AP/Deo Stick Formulierungen wurden von einem trainierten Geruchspanel (4 Personen) untersucht. Dabei wurde der Geruch nach dem Schulnotensystem von 1 (sehr gut) bis 6 (sehr schlecht) bewertet. Die erste Geruchsbewertung der Sticks 2A und 2B fand einen Tag nach Herstellung, die zweite vier Wochen nach Lagerung in einer Klimakammer bei 22 °C statt:

| | 1. Bewertung | 2. Bewertung |
|---|---|---|
| Stick 2A | 2,0 | 2,5 |
| Stick 2B | 2,5 | 3,5 |

Überraschenderweise wurde weiterhin festgestellt, dass der Stick 2A auf schwarzem Baumwollgewebe einen geringfügig geringeren weißen Abrieb zeigte als der Stick 2B. Der Weißelgrad ist mithin gegenüber einer Standardfomulierung überraschenderweise vermindert.

## Patentansprüche

1. Kosmetische und/oder dermatologische Zubereitungen hergestellt unter Verwendung von Polyetheralkoholen, **dadurch gekennzeichnet, dass** zur Herstellung der Polyetheralkohole
a) ein DMC-Katalysator verwendet wird und
b) die so erhaltenen Polyetheralkohole nicht weiter behandelt werden.

2. Kosmetische und/oder dermatologische Zubereitungen gemäß Anspruch 1, **dadurch gekennzeichnet, dass** ein DMC-Katalysator in einer Konzentration kleiner 1000 wppm verwendet wird.

3. Kosmetische und/oder dermatologische Zubereitungen gemäß zumindest einem der Ansprüche 1 bis 2, **dadurch gekennzeichnet, dass** es sich bei den Polyetheralkoholen um reine Polypropylenglycolalkylether handelt.

4. Kosmetische und/oder dermatologische Zubereitung gemäß zumindest einem der Ansprüche 1 bis 3, **dadurch gekennzeichnet, dass** ein oder mehrere Polyetheralkohole der Formel (I) enthalten sind,
R¹-[O-(A)ₙ-(B)ₘ-H]ₓ (I)
wobei
R¹ ein linearer oder verzweigter Alkylrest mit 3 bis 22 C-Atomen ist,
A unabhängig von B eine gleiche oder unterschiedliche Ethylenoxy-, Propylenoxy-, Butylenoxy-, Styroloxy-, Cyclohexyloxyeinheit oder eine durch Epoxidringöffnung aus einer Glycidylverbindung hervorgegangene Einheit ist,
B unabhängig von A eine gleiche oder unterschiedliche Ethylenoxy-, Propylenoxy-, Butylenoxy-, Styroloxy-, Cyclohexyloxyeinheit oder eine durch Epoxidringöffnung aus einer Glycidylverbindung hervorgegangene Einheit ist,
m gleich 0 bis 20,
n gleich 1 bis 40,
x eine ganze Zahl von 1 bis 6 ist
und die Monomereinheiten A und B in ihrer Abfolge beliebig sowohl blockartig als auch statistisch aneinandergereiht sein können.

5. Kosmetische und/oder dermatologische Zubereitungen gemäß zumindest einem der Ansprüche 1 bis 4, **dadurch gekennzeichnet, dass** es sich bei den Polyetheralkoholen um PPG-3 Myristylether, PPG-11 Stearylether, PPG-14 Butylether oder PPG-15 Stearylether handelt.

6. Kosmetische und/oder dermatologische Zubereitungen gemäß zumindest einem der Ansprüche 1 bis 5, **dadurch gekennzeichnet, dass** eine zusätzliche Komponente enthalten ist ausgewählt aus der Gruppe der Emollients, Emulgatoren und Tenside, Verdicker, Viskositätsregler, Stabilisatoren, UV-Lichtschutzfilter, Antioxidantien und Vitamine, Hydrotrope oder Polyole, Fest- und Füllstoffe, Filmbildner, Perlglanzadditive, Deodorant- und Antitranspirantwirkstoffe, Esteraseinhibitoren, Insektrepellentien, Selbstbräuner, Konservierungsstoffe, Konditioniermittel, Parfüme, Farbstoffe, Biogene Wirkstoffe, Pflegeadditive, Überfettungsmittel und/oder Lösungsmittel.

7. Kosmetische und/oder dermatologische Zubereitung nach einem oder mehreren der vorhergehenden Ansprüche, **dadurch gekennzeichnet, dass** die Zubereitungen dickflüssig bis halbfest und bis hin zu cremefest sind und in einem für kosmetische Stifte geeigneten oder Deoroller-Behältnis oder einem Zerstäuberpumpen-Behältnis an den Verbraucher weitergegeben werden.

8. Kosmetische und/oder dermatologische Zubereitung nach einem oder mehreren der vorhergehenden Ansprüche, **dadurch gekennzeichnet, dass** die Zubereitung neben anderen kosmetischen Zusatzstoffen Parfüm und/oder Duftstoffe enthält.

9. Verwendung einer Antitranspirantformulierung nach mindestens einem der vorhergehenden Ansprüche zur Auftragung auf die menschliche Haut, insbesondere zur Verminderung der Schweissbildung.
